# EUROPEAN PATENT APPLICATION

(11) **EP 1 495 738 A1**
(43) Date of publication of application: **12.01.2005**
(21) Application number: 04103231.9
(22) Date of filing: 08.07.2004
(51) Int. Cl.: A61F 7/10

(54) **Cooling device for pain refief**

(30) Priority: 08.07.2003 GB 0315974
(71) Applicant: COOLANALGESIA LIMITED, Cambridge, Cambridgeshire CB2 1NT (GB)
(72) Inventor: McMahon Dr., Richard, Cambridgeshire CB5 9JG (GB)
(74) Representative: Brunner, Michael John

(57) **Abstract**

A cooling device is disclosed for application to the skin of a patient to relieve pain during a medical procedure. The device has an annular cooling contact element for application to the skin and a cooling means is disposed laterally of the contact element, to one side thereof. A heat flow path extends laterally from the contact element, transversely of the axis of the contact element, to the cooling means, so that in use heat is withdrawn from the contact element to the cooling means. A temperature detector is disposed to detect temperature within the envelope of the annular cooling contact element.

## Description

The present invention relates to a cooling device for application to the skin of a patient to relieve pain during a medical procedure or the like.

Cooling is known to relieve pain in a patient's skin and various devices have been developed for relieving pain during laser skin treatment based on this principle (see for example US-A-6264649). Anaesthesia, or pain relief, by cooling, avoids the need for a general anaesthetic, with its attendant risk and prolonged recovery period, or local anaesthetic which can be painful to administer and is not particularly effective. More recently, cooling has been proposed for relieving pain during non-laser skin treatments, for procedures such as Botox injections, and the taking of biopsies.

It is known, for example, to place a cooled material which is transparent to laser radiation in contact with the skin over the treatment area such as in US-A-5486172, EP-A-0783904 and EP-A-0827716. To ensure good lateral transfer of heat, a material of high thermal conductivity is needed for the transparent window. Transparent materials with a relatively high thermal conductivity include sapphire, quartz and diamond. The transparent material can be cooled peripherally, either by a flow of chilled fluid as described by in EP-A-0783904 or by thermoelectric cooling, as described in EP-A-0827716. However, there are drawbacks with such a device, which include the lack of means for moving skin debris, the opaqueness of sapphire to certain wavelengths, such as the radiation from a CO₂ laser and the thermal properties of sapphire are also such that effective cooling may not be possible because the thermal conductivity is not sufficiently high to enable really rapid removable of heat from the sapphire. Furthermore, this method of cooling is impracticable where physical access to the skin is required, e.g. for injections.

In US-A-5486172 a cell with upper and lower transparent plates, with the lower plate in contact with the skin is employed. Cooling fluid is passed through the cell to cool the plate in contact with the skin, thereby cooling the skin itself. Although this approach gives better heat transfer than in the peripherally cooled window, the other drawbacks remain.

Methods of cooling the area of skin to be treated without physical contact have been devised. One method involves spraying the skin with a refrigerant that evaporates at room temperature and pressure, as described by in US-A- 5814040. Although a large degree of skin cooling is attainable by this method, it is difficult to control the degree and uniformity of cooling, which is a particular disadvantage if cooling is to be maintained for say a second or longer. Alternatively cooling can be achieved by using chilled air as in the apparatus sold by Zimmer Elektromedezin or as proposed in US-A-6475211.

In our US-A-6264649, mentioned above, we proposed another approach to inducing anaesthesia by cooling, namely the concept of a ring or field block induced by the cooling. In this approach, the zone around the area to be treated is cooled, for example by contact cooling with a hollow metal body through which a coolant is circulated. The advantage of this approach is that the treatment area can be open, allowing all wavelengths of radiation to reach the treatment site unattenuated and allowing the removable of debris. Open access also allows the possibility of using other procedures, including injections and biopsies. The CoolAnalgesia C300A product is designed for attachment to a laser, cooling being achieved by circulation of a chilled fluid from the chiller unit.

Paradigm Trex has manufactured a hand-held annular cooling device as a variant of their sapphire window device which has a contact cooling plate in the form of a sapphire plate extending into a chamber. A cryogen spray is then directed onto the part of sapphire plate within the chamber to cool the plate. The temperature of the plate within the chamber is detected and controlled by regulation of the flow of cryogen. However, such a device has short-comings because although the thermal conductivity of sapphire is high for a non-metal it is low compared to say copper (just over 12%), so that, with the geometry used for the device, there is a substantial thermal gradient along the sapphire when extracting the energy needed to achieve significant cooling of the skin. An annular cooling device of inside diameter 15 mm and outside diameter 40 mm has a contact area of 10.8 cm², corresponding to an approximate power flow between 25 and 100 W depending on the patient and the area of the body being treated. If this is to be transmitted laterally through a sapphire bar of cross section 40 by 5 mm to a cooling source, the temperature drop would be up to 117 degrees C/cm, which is impractical. In practice the rate of achievable heat transfer, and hence skin cooling would be much lower. If the bar were of copper, the temperature drop would be 14 degrees C/cm. These results emphasize the practical difficulty of transmitting heat laterally by thermal conduction and the consequent substantial differences in temperature between the cooling source and the surface contacting the skin. The unpredictability of the temperature of the cooling surface will lead to an uncertainty in the actual degree of cooling achieved and hence poor pain control.

According to the present invention there is provided a cooling device for application to the skin of a patient to relieve pain during a medical procedure or the like, the device including
a substantially annular cooling contact element for application to the skin;
a cooling means disposed laterally of the contact element, to one side thereof;
a heat flow path extending laterally from the contact element, transversely of the axis of the contact element, to the cooling means, whereby in use heat is withdrawn from the contact element to the cooling means; and,
a temperature detector disposed to detect temperature within the envelope of the annular cooling contact element.

The amount of heat to be extracted from the skin to achieve a desired degree of cooling can be estimated from a knowledge of the thermal properties of skin, namely the specific heat capacity and thermal conductivity. In a simple model the skin can be taken as a uniform semi-infinite solid and the cooling element as a heatsink of constant temperature in series with a specific thermal resistance per unit area. Heat transfer under these conditions is governed by the well-known diffusion equation. Once the cooling element is placed in contact with the skin, the surface temperature falls as heat is extracted. Heat then diffuses to the surface of the skin and the cooled region penetrates into the body. The rate of heat extraction is highest on initial contact and then falls progressively as contact is maintained. Although the rate of heat extraction is changing constantly, representative values for contact on a time scale of seconds are 5 to 10 W/cm² for a contact temperature of -6 degrees C.

Preferably, the temperature detector is attached to the cooling plate and comprises, for example, a thermocouple, platinum resistance element, or a thermistor, but, alternatively, the temperature detector may comprise an infrared detector having a focal point or detecting spot within the aperture formed in the annular cooling contact element.

An electronic control circuit is provided to control the flow of cryogen or other cooling fluid into the cooling means by varying the flow with an electrically controlled valve or alternatively with a pump, for example a peristaltic pump. The electronic circuit acts to regulate the flow to maintain the temperature of the cooling element constant. This can be achieved by analogue or digital means. A second temperature sensor may be provided to act as a backup in order to provide a safety feature to take over control in the event of a failure of the normal sensor temperature. This ensures that the temperature of the contact element does not fall to a level at which frostbite or other skin damage might occur.

In this specification although the contact element is referred to as "annular" the term is intended to include rings and the like which may be split-rings, i.e. the ring being not quite completely circular having a small gap between ends of the annular arc.

Preferably, the annular cooling contact element comprises a metallic ring, for example of copper, and the heat flow path may also comprise a metal (e.g. copper again) component, extending to a cooling means in the form of a chamber within which a cryogen is applied to the end of the heat flow path within the chamber. Alternatively, the annular cooling contact element may be hollow and a cryogen or other heat carrying fluid may flow through the contact element and along a passage forming the heat flow path, to a heat exchanger within the cooling means. In this arrangement cryogen may be supplied via a supply path parallel to the heat flow path into the annular cooling contact element and evaporated in passages within the contact element, exhaust cryogen gas, passing through the heat flow path to the cooling means. The control of cryogen evaporation may be achieved by feedback from the temperature detector or by multiple detectors on the annular cooling contact element by means of a skin temperature detector.

In a further construction, a secondary circuit may be provided to circulate fluid through the cooling contact element and the heat flow path to a heat exchanger within the cooling means and within which a cryogen is used to cool the fluid passing through the contact element and the heat flow path. It may be possible to use a Peltier cooler within the cooling means, and heat pipe type solutions might also be possible, but in all cases, it is desirable to control the temperature of the annular cooling contact element by means of feedback from the temperature detector.

In a further approach, contact cooling may be augmented by directfluid cooling onto the treated area, as disclosed in our US-A-6264649. In the present case, directing a separately controlled cryogen spray onto the skin is a convenient means of achieving this.

An example of a cooling device constructed in accordance with the present invention will now be described with reference to the accompanying drawings in which:
Figure 1 is an exploded isometric view of a first example;
Figure 2 is an isometric view of the first example after assembly;
Figure 3 is an exploded isometric view of a second example;
Figure 4 is an isometric view of the second example as assembled;
Figure 5 is an exploded isometric view of a third example;
Figure 6 is an isometric view of the third example as assembled; and
Figure 7 shows, diagrammatically, the connection of a temperature detector to a control circuit for any of the examples above.

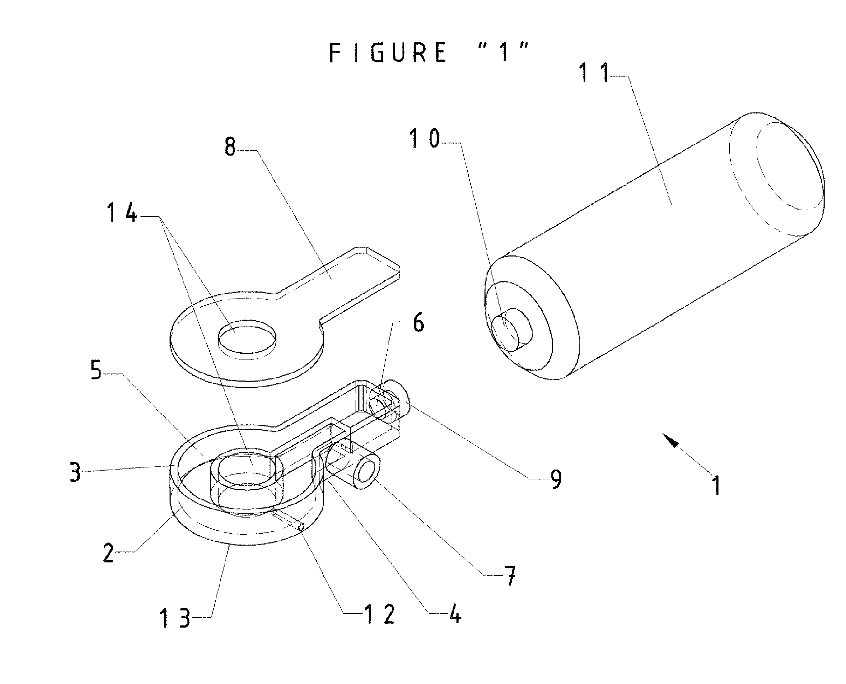

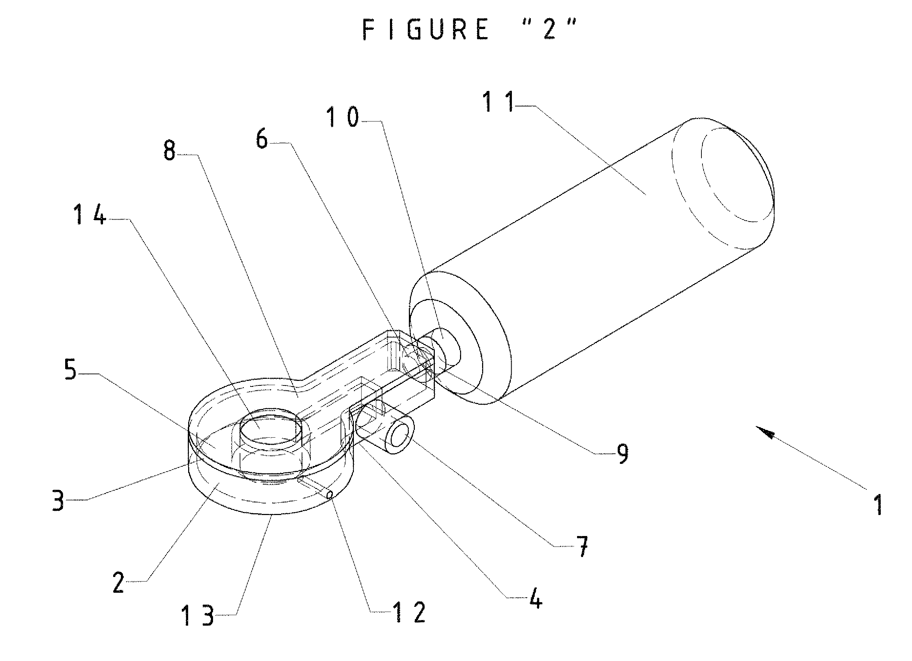

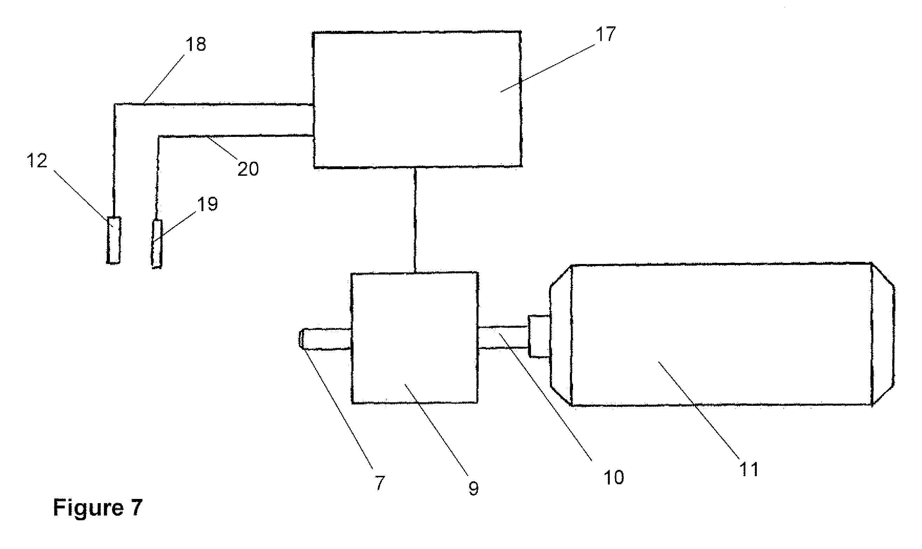

Figures 1 and 2 show a first cooling device 1 according to the invention, the device including an annular contact element 2 in the form of a hollow annular housing 3 with a transverse extension 4, defining a heat flow path 5 from an inlet end 6 to an exhaust port 7, closed by a correspondingly shaped cover 8. The inlet end 6 of the heat flow path 5 has a solenoid valve 9 to which is attached, via a connector 10, a cylinder 11 of a liquid cryogen. A temperature detector 12, in the form of a thermocouple, is disposed and embedded in the lower surface 13 of the annular contact element 3 and is connected via a suitable connection 18 (see Figure 7) to provide feedback to a control circuit 17 (see Figure 7) which is arranged to operate the valve 9 so as to maintain the surface 13 at a desired cooling temperature.

The contact element 2 is formed of a suitable metal (eg. copper) integrally with the extension 4 and a central aperture 14 provides for visual inspection of the patient's skin during a surgical technique and also allows for the removal of skin debris by, for example, the application of suction through the aperture.

In the first example, the cryogen is able to flow through the inlet valve 9, along the heat flow path 5 around the annular contact element 2, back along the transverse extension 4 and out through the exhaust port 7.

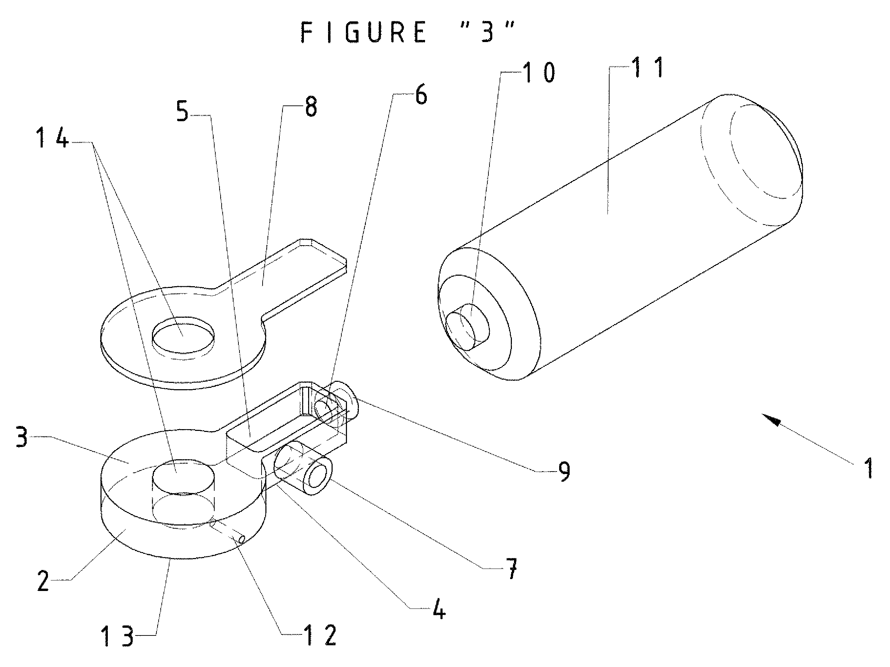

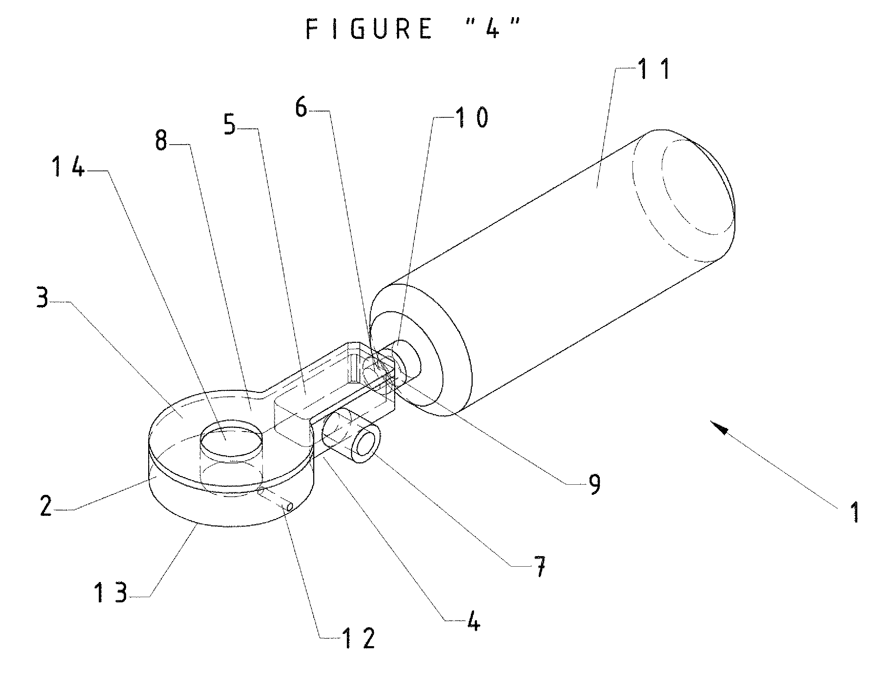

Figures 3 and 4 show a second example, similar to the first and with similar reference numerals used for easier reference, but in this case, the contact element 2 comprises a solid copper annulus integrally formed with a hollow transverse extension 4 with an elongate heat flow path 5 so that cryogen is applied through the flow path 5 and out via the exhaust port 7, maintaining the temperature of the solid contact element 2 as before.

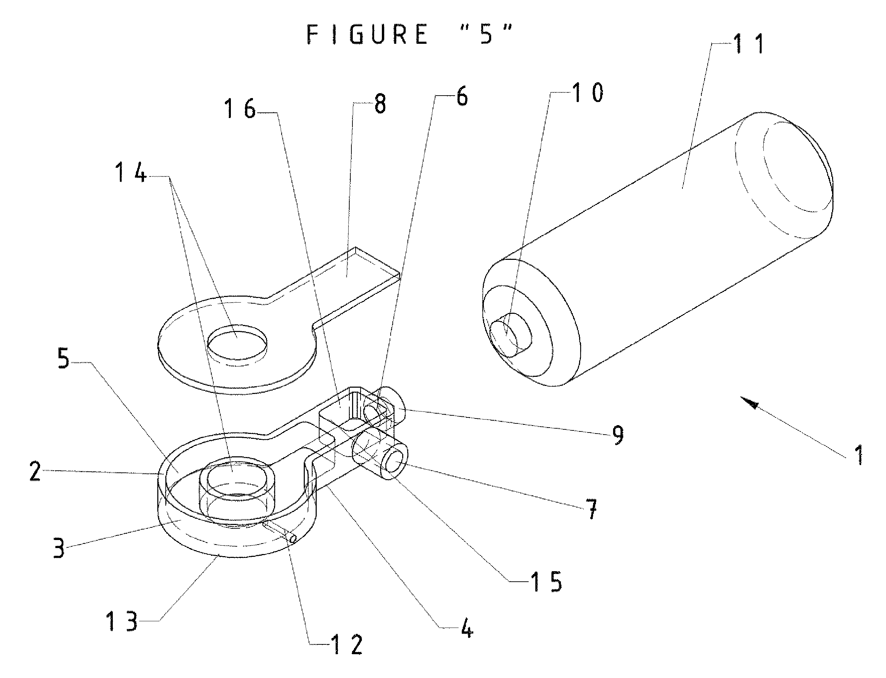

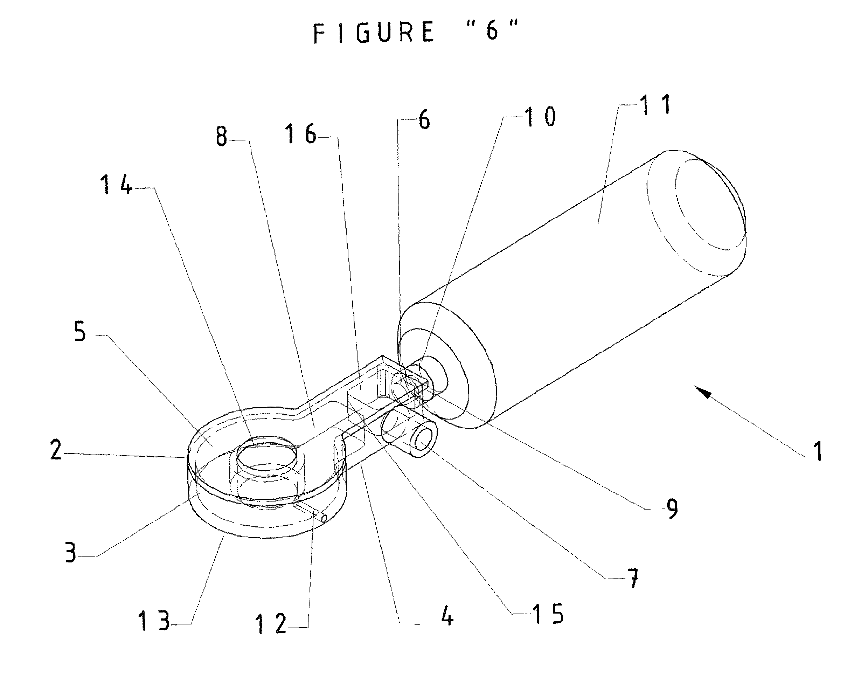

A further example is shown in Figures 5 and 6 and in this case, as in the case of the first example, there is a hollow contact element 2 having a housing 3 with a substantially annular chamber 5 providing a secondary cooling cryogen chamber in order to cool the surface 13 of the contact element 2, the chamber 5 being in contact with a wall 15, and through the wall with a primary cooling chamber 16 through which cryogen flows through the valve 9 and out via the exhaust port 7 as before. In this case, the heat flow path extends laterally but includes conduction through the wall 15.

Figure 7 shows, diagrammatically, the connection of the temperature detector 12 to the control circuit 17 and the connection of the control circuit to the solenoid value 9 controlling the flow of the cryogen from the cylinder 11.

The figure also shows the secondary, safety temperature detector 19, connected to the control circuit 17 via connection 20. The control circuit is arranged to interpret an out-of-range (ie too cold) temperature from the detector 19 as an error in the temperature being reported from the first detector 12 and shuts down the valve 9 to prevent the further flow of cryogen through the valve.

## Claims

1. A cooling device for application to the skin of a patient to relieve pain during a medical procedure or the like, the device including
a substantially annular cooling contact element for application to the skin;
a cooling means disposed laterally of the contact element, to one side thereof;
a heat flow path extending laterally from the contact element, transversely of the axis of the contact element, to the cooling means, whereby in use heat is withdrawn from the contact element to the cooling means; and,
a temperature detector disposed to detect temperature within the envelope of the annular cooling contact element.

2. A device according to claim 1, wherein the temperature detector is attached to the cooling plate and comprises one of a thermocouple, platinum resistance element, or a thermistor.

3. A device according to claim 1, wherein the temperature detector comprises an infrared detector having a focal point or detecting spot within the aperture formed in the annular cooling contact element.

4. A device according to any of claims 1 to 3, wherein the annular cooling contact element comprises a metallic ring.

5. A device according to any of claims 1 to 4, wherein the heat flow path comprises a metal component, extending to a cooling means in the form of a chamber within which a cryogen is applied to the end of the heat flow path within the chamber.

6. A device according to any of claims 1 to 3, wherein the annular cooling contact element is hollow and a cryogen or other heat carrying fluid is provided to flow through the contact element and along a passage forming the heat flow path, to a heat exchanger within the cooling means.

7. A device according to claim 6, wherein a supply path for cryogen is provided parallel to the heat flow path and, in use, cryogen is evaporated in passages within the contact element, exhaust cryogen gas passing through the heat flow path to the cooling means.

8. A device according to claim 7, further including a control circuit for controlling cryogen evaporation by means of feedback from the temperature detector.

9. A device according to any of claims 1 to 8, wherein a secondary circuit is provided to circulate fluid through the cooling contact element and the heat flow path to a heat exchanger within the cooling means and within which a cryogen is used to cool the fluid passing through the contact element and the heat flow path.
